# EUROPEAN PATENT APPLICATION

(11) **EP 0 982 028 A1**
(43) Date of publication of application: **01.03.2000**
(21) Application number: 98115650.8
(22) Date of filing: 20.08.1998
(51) Int. Cl.: A61K 31/335

(54) **New use of taxoid derivatives**

(71) Applicant: RHONE-POULENC RORER S.A., 92160 Antony (FR)
(72) Inventor: Bissery, Marie-Christine, 94400 Vitry sur Seine (FR); Vrignaud, Patricia, 77380 Combs la Ville (FR); Roberts, Simon, Harlow, Essex CM17 9PG (GB); Brealey, Clive, Wiggington, Tring, Herts HP23 6HE (GB)
(74) Representative: Le Pennec, Magali

(57) **Abstract**

The present invention relates a new use of taxoid derivatives. It relates more precisely to a method for treating abnormal cell proliferation of different resistant cell lines expressing multidrug resistance P-glycoprotein. This type of cells are representative of colon cancer cells. The product of the present invention are also usable to treat the colon cancer of mammals including men.

## Description

The present invention relates a new use of taxoid derivatives. It relates more precisely to a method for treating abnormal cell proliferation of different resistant cell lines expressing multidrug resistance P-glycoprotein. This type of cells are representative of colon cancer cells. The present invention is related to the treatment of abnormal cell proliferation of different resistant cell lines expressing multidrug resistance P-glycoprotein and is also related to the treatment of abnormal cell proliferation of different resistant cell lines expressing multidrug resistance P-glycoprotein and is also related to the treatment of colon tumors of mammals including men by administrating a compound of general formula (I) or a pharmaceutically acceptable salt or solvante thereof : 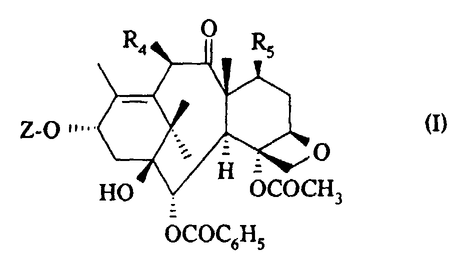 in which :
Z represents a hydrogen atom or a radical of general formula : 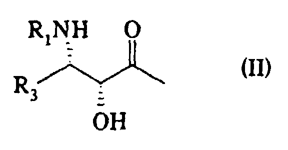 in which :
R₁ represents
a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals,
a thenoyl or furoyl radical or
a radical R₂-O-CO- in which R₂ represents :
   - an alkyl radical containing 1 to 8 carbon atoms,
   - an alkenyl radical containing 2 to 8 carbon atoms,
   - an alkynyl radical containing 3 to 8 carbon atoms,
   - a cycloalkyl radical containing 3 to 6 carbon atoms,
   - a cycloalkenyl radical containing 4 to 6 carbon atoms or
   - a bicycloalkyl radical containing 7 to 10 carbon atoms,
   these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
   - a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or
   - a 5-membered aromatic heterocyclic radical preferably chosen from furyl and thienyl radicals,
   - or a saturated heterocyclic radical containing 4 to 6 carbon atoms, optionally substituted with one or more alkyl radicals containing 1 to 4 carbon atoms,
R₃ represents
an unbranched or branched alkyl radical containing 1 to 8 carbon atoms,
an unbranched or branched alkenyl radical containing 2 to 8 carbon atoms,
an unbranched or branched alkynyl radical containing 2 to 8 carbon atoms,
a cycloalkyl radical containing 3 to 6 carbon atoms,
a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals,
on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
R₄ represents
an alkoxy radical containing 1 to 6 carbon atoms in an unbranched or branched chain,
an alkenyloxy radical containing 3 to 6 carbon atoms in an unbranched or branched chain,
an alkynyloxy radical containing 3 to 6 carbon atoms in an unbranched or branched chain,
a cycloalkyloxy radical containing 3 to 6 carbon atoms or
a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
R₅ represents
an alkoxy radical containing 1 to 6 carbon atoms in an unbranched or branched chain,
an alkenyloxy radical containing 3 to 6 carbon atoms,
an alkynyloxy radical containing 3 to 6 carbon atoms,
a cycloalkyloxy radical containing 3 to 6 carbon atoms or
a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

Preferably, the aryl radicals which can be represented by R₃ are phenyl or α-or β-naphthyl radicals optionally substituted with one or more atoms or radicals chosen from halogen atoms (fluorine, chlorine, bromine, iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, on the understanding that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals.

Preferably, the heterocyclic radicals which can be represented by R₃ are 5-membered aromatic heterocyclic radicals containing one or more identical or different atoms chosen from nitrogen, oxygen and sulphur atoms, optionally substituted with one or more identical or different substituents chosen from halogen atoms (fluorine, chlorine, bromine, iodine) and alkyl radicals containing 1 to 4 carbon atoms, aryl radicals containing 6 to 10 carbon atoms, alkoxy radicals containing 1 to 4 carbon atoms, aryloxy radicals containing 6 to 10 carbon atoms, amino radicals, alkylamino radicals containing 1 to 4 carbon atoms, dialkylamino radicals in which each alkyl portion contains 1 to 4 carbon atoms, acylamino radicals in which the acyl portion contains 1 to 4 carbon atoms, alkoxycarbonylamino radicals containing 1 to 4 carbon atoms, acyl radicals containing 1 to 4 carbon atoms, arylcarbonyl radicals in which the aryl portion contains 6 to 10 carbon atoms, cyano, carboxyl or carbamoyl radicals, alkylcarbamoyl radicals in which the alkyl portion contains 1 to 4 carbon atoms, dialkylcarbamoyl radicals in which each alkyl portion contains 1 to 4 carbon atoms or alkoxycarbonyl radicals in which the alkoxy portion contains 1 to 4 carbon atoms.

Preferably, the radicals R₄ and R₅, which may be identical or different, represent unbranched or branched alkoxy radicals containing 1 to 6 carbon atoms, optionally substituted with a methoxy, ethoxy, ethylthio, carboxyl, methoxycarbonyl, ethoxycarbonyl, cyano, carbamoyl, N-methylcarbamoyl, N-ethylcarbamoyl, N,N-dimethylcarbamoyl, N,N-diethylcarbamoyl, N-pyrrolidinocarbonyl or N-piperidinocarbonyl radical.

More specially, the present invention relates to the products of general formula (I) in which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl (methyl), alkoxy (methoxy), dialkylamino (dimethylamino), acylamino (acetylamino), alkoxycarbonylamino (tert-butoxycarbonylamino) or trifluoromethyl radicals, or a 2- or 3-furyl, 2- or 3-thienyl or 2-, 4- or 5-thiazolyl radical, and R₄ and R₅, which may be identical or different, each represent an unbranched or branched alkoxy radical containing 1 to 6 carbon atoms.

Still more specially, the present invention relates to the products of general formula (I) in which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R₄ and R₅, which may be identical or different, each represent a methoxy, ethoxy or propoxy radical.

Of even more special interest are the products of general formula (I) in which R₃ represents a phenyl radical and R₁ represents a ter-butoxycarbonyl radical, R₄ and R₅, which may be identical or different, represent a methoxy, ethoxy or propoxy radical.

In a still higher interest, the present invention relates to 4α-acetoxy-2α-benzoyloxy-5β,20-epoxy-1β-hydroxy-7β,10β-dimethoxy-9-oxo-11-taxen-13α-yl (2R,3S)-3 tert-butoxycarbonylamino-2-hydroxy-3-phenylpropionate of formula (Ia)

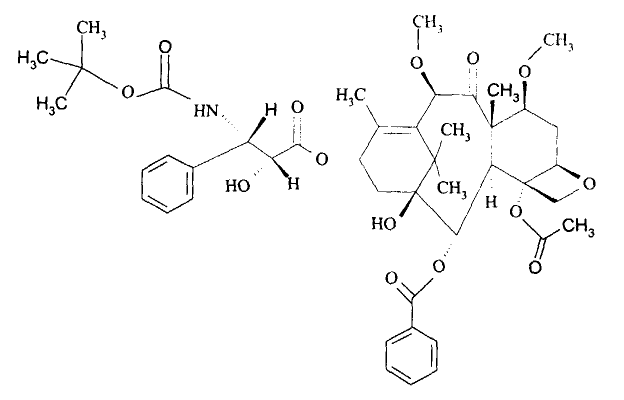

It is known, from patent WO 96/30355, to prepare a derivative according to the present invention by two processes. According to a first, multi-step process, starting with 10-deacetylbaccatin III of formula : 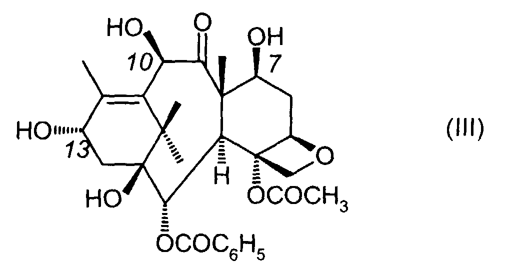 it is selectively protected in positions 7 and 13, for example in the form of a silyl diether, followed by the action of a product of general formula:

R-X (IV)

in which R represents a radical as defined above and X represents a reactive ester residue such as a sulphuric or sulphonic ester residue or a halogen atom, to give a product bearing the unit -OR in position 10 and silyl groups in positions 7 and 13. Next, the silyl protecting groups are replaced with hydrogen atoms to give a compound still bearing the group -OR in position 10 and OH groups in positions 7 and 13. The latter derivative is etherified selectively in position 7 by reaction with the derivative of formula IV to give the derivative of formula (I) in which Z is equal to hydrogen.

The final step consists in esterifying in position 13, according to a process which is known per se, the derivatives of formula (Ia), in which Z represents hydrogen, in the presence of a β-lactam according, for example, to the process described in patent EP 617,018, or in the presence of an oxazolidine as described, for example, in patent WO 96/30355 mentioned above. After deprotection of the protecting groups in positions 7 and 10, an ester of formula (Ia) is thus obtained in which Z is other than hydrogen and R represents hydrogen. The next step consists in reacting the positions 7 and 10 simultaneously by the action of a reagent formed in situ from a sulphoxide of formula (V) and acetic anhydride (Pummerer-type reaction),

R-SO-R (V)

in which R has the same meaning as above, to form an alkylthioalkyloxy-type intermediate on positions 7 and 10.

The final step, which allows the desired compound of formula (Ia) to be obtained, is carried out on the intermediate compound obtained above, by the action of activated Raney nickel.

Generally, the action of the reagent formed in situ from sulphoxide of general formula (V), preferably dimethyl sulphoxide and acetic anhydride, is carried out in the presence of acetic acid or an acetic acid derivative such as a haloacetic acid, at a temperature of between 0 and 50°C.

Generally, the action of the activated Raney nickel in the presence of an aliphatic alcohol or an ether is carried out at a temperature of between -10 and 60°C.

In the FR 97-14442 application a further process has been described. This invention allows, in a single step, the direct, selective and simultaneous alkylation of the two hydroxyl functions in positions 7 and 10 of 10-deacetylbaccatin or of derivatives there of esterified in position 13, of formula (VI) 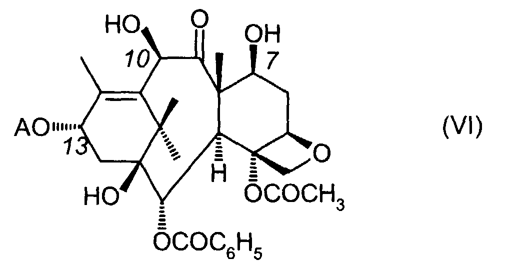 in which A represents hydrogen or a side chain of formula (IIa) below : 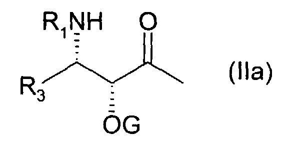 in which G represents a protecting group for the hydroxyl function, R₁ and R₃ have the same meaning as in formula (II)
or an oxazolidine unit of formula (Id) : 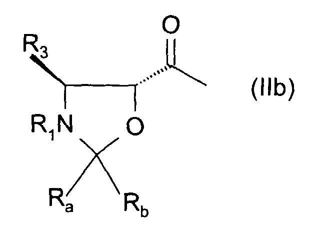 in which R₁ and R₃ have the same meaning as in formula (II), Rₐ and R_{b}, which may be identical or different, represent hydrogen or alkyl, aryl, halo, alkoxy, arylalkyl, alkoxyaryl, haloalkyl, haloaryl, it being possible for the substituents optionally to form a 4- to 7-membered ring.

It is preferred to use 10-deacetylbaccatin as starting material, i.e. the product of formula (III), which allows appreciable economy as regards the process and moreover avoids the intermediate protection and deprotection steps necessary in the old processes.

Among the groups G for protecting the hydroxyl function of formula (IIa), it is generally preferred to choose all of the protecting groups described in books such as Greene and Wuts, Protective Groups in Organic Synthesis, 1991, John Wiley & Sons, and MacOmie, Protective Groups in Organic Chemistry, 1975, Plenum Press, and which are deprotected under conditions which degrade the rest of the molecule little or not at all, such as, for example:
- ethers, and preferably ethers such as methoxymethyl ether, 1-ethoxyethyl ether, benzyloxymethyl ether, p-methoxybenzyloxymethyl ether, benzyl ethers optionally substituted with one or more groups such as methoxy, chloro, nitro, 1-methyl-1-methoxyethyl ether, 2-(trimethylsilyl)ethoxymethyl ether, tetrahydropyranyl ether and silyl ethers such as trialkylsilyl ethers,
- carbonates such as trichloroethyl carbonates.

More particularly, the radicals Rₐ and R_{b} of general formula (IIb) are chosen from those described in patent WO 94/07878 and the derivatives more particularly preferred are those in which Rₐ is hydrogen and R_{b} is a p-methoxyphenyl radical.

The alkylating agent is chosen from :
- alkyl halides, and preferably from alkyl iodides (RI)
- alkyl sulphates such as methyl sulphate
- oxoniums such as trialkyloxonium boric salts, in particular trimethyloxonium tetrafluoroborate (Me₃OBF₄).

Methyl iodide is preferably used.

The alkylating agent is used in the presence of an anionization agent such as one or more strong bases, in anhydrous medium.

Among the bases which can be used in anhydrous medium, mention may be made of :
- alkali metal hydrides such as sodium or potassium hydride
- alkali metal alkoxides such as potassium tert-butoxide
- silver oxide Ag₂O
- 1,8-bis(dimethylamino)naphthalene
- mono- or dimetallic base mixtures such as those described, for example, in publications such as P. Caubère Chem. Rev. 1993, 93, 2317-2334 or M. Schlosser Mod. Synth. Methods (1992), 6, 227-271; in particular the alkyllithium/alkali metal t-butoxide or alkali metal amide/alkali metal t-butoxide combinations are preferred. One of the two bases can be generated "in situ".

Among all of the possible combinations of alkylating agent and anionization agent, it is preferred to use methyl iodide in the presence of potassium hydride.

The reaction is preferably carried out in an organic medium which is inert under the reaction conditions. Among the solvents, it is preferred to use:
- ethers such as tetrahydrofuran or dimethoxyethane
- when silver oxide is used, it is preferred to use polar aprotic solvents such as dimethylformamide, or aromatic solvents such as toluene
- when 1,8-bis(dimethylamino)naphthalene is used, it is preferred to use alkylesters such as ethylacetate.

For better implementation of the invention, it is preferred to use a molar ratio between the anionization agent and the substrate of greater than 2 and preferably between 2 and 20.

It is also preferred to use a molar ratio between the alkylating agent and the substrate of greater than 2 and preferably between 2 and 40.

It is preferred to use a reaction temperature of between -30°C and 80°C.

The reaction time advantageously ranges between a few hours and 48 hours depending on the reagents chosen.

After the alkylating step, when the latter is carried out on 10-deacetylbaccatin, the process then proceeds, in a known manner, to the esterification step according, for example, to the processes described in patents EP 617,018 or WO 96/30355 mentioned above.

Thus, according to a first, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with a suitably protected β-lactam in the presence of an activating agent chosen from tertiary amines and metal bases which ensure the formation of an alkoxide in position 13. Deprotection of the side chain is then achieved by the action of an inorganic or organic acid.

Thus, according to a second, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with an oxazolidine in the presence of a coupling agent such as diimides in the presence of an activating agent such as dialkylaminopyridines. Opening of the oxazolidine is achieved by the action of an inorganic or organic acid.

According to a third process, the procedure begins with the esterification in position 13 of baccatin suitably protected in positions 7 and 10, with a β-lactam or an oxazolidine in the presence of a coupling agent and/or an activating agent as described in the above two processes. After deprotection in positions 7 and 10, the dietherification in positions 7 and 10 is carried out by an alkylating agent in the presence of a strong base. Deprotection of the side chain is then achieved by the action of an inorganic or organic acid.

The products of general formula (I) have remarkable biological properties.

In vivo, the products of general formula (I) proved active in mice grafted with the colon adenocarcinoma C51 or C38 at doses of between 1 and 30 mg/kg, as well as on other liquid or solid tumours.

The compounds of formula I have anti-tumor properties, more particularly, activity against tumors which are resistant to Taxol® and Taxotere®. Such tumors include, for example, colon tumors which have an elevated expression of multidrug resistance P-glycoprotein. Multi-drug resistance is the usual term relating to the resistance by a tumor against various compounds having differing structures and mechanisms of action. Taxoids are generally known to be highly recognized by experimental tumors such as P388/DOX, a P388 murine leukemia cell line selected for doxorubicin (DOX) resistance, which express P-glycoprotein. The compounds according to the present invention are less recognized by P388/DOX. More particularly, the compounds are less recognized than Taxotere®.

The compounds of formula (I) are mainly used for preparing a medecine for treating abnormal cell proliferation of cell lines expressing the multidrug resistance P-glycoprotein. The compound of formula I are mainly used for preparing a medecine for treating colon cancer.

The compound and mainly compound fo formula (I) where R₄ and R₅ are each methoxy has the property to be active compared to the other known taxoids such as Taxol® or Taxotere® to treat the cancer cells lines expressing the multidrug resistance P-glycoprotein. It is also active against tumor cells resistant to Doxorubicine or resistant to Vincristine

The product of formula (I) can be used concurrently with at least other therapeutic treatment. It is more preferably used with other therapeutic treatment comprising antineoplastic drugs, monoclonal antibodies, immunotherapies, radiotherapies, or biological response modifiers.

The product of formula (I) is preferably administered by parenteral administration such as intravenous, intraperitoneal, intramuscular or subcutaneous administration.

### Example

### 1. INTRODUCTION

Product of formula (Ia) is a potent anti-cancer agent in pre-clinical models.

### In vitro antiproliferative properties on resistant cell lines

Docetaxel was found to be cross-resistant to cell lines expressing the multidrug resistance P-glycoprotein (RIGEL I. and HORWITZ S.B., Studies with RP 56976 (Taxotere®: a semisynthtetic analogue of Taxol, J. Natl. Cancer Inst., **83**, 288-291, 1991).

The cytotoxicity of product Ia was examined against different resistant cell lines expressing the multidrug resistance P-glycoprotein, in comparison with docetaxel after 4 days of continuous exposure using standard methods.

The resistance factor was calculated by dividing the IC₅₀ value obtained on resistant cells by the IC₅₀ value obtained on sensitive cells.

Product of formula Ia was found more active than docetaxel on all the resistant tumor cell lines which have been evaluated. Interestingly, product of formula Ia was found minimally cross-resistant (resistance factor: 1.8 to 4.3) in the P388/TXT, P388/VCR, HL60/TAX and Calc18/TXT cell lines that are moderately cross-resistant to docetaxel (resistance factor: 4.8 to 23.5) and which could be more representative of the clinical situation.

In addition, a strong decrease of the cross-resistance for product of formula Ia (resistance factor: 7.6 and 10) was found in the two most resistant to docetaxel cell lines KB VI and P388/DOX (resistance factor: 59 and 50).

Product of formula Ia was also evaluated on the murine P388/CPT5 cell line with acquired resistance to camptothecin that do not express the P-glycoprotein. A strong decrease in the cross-resistance was found for product of formula Ia and docetaxel (resistance factor: 4.8 and 10.5) as compared to camptothecin (resistance factor: 286). This result suggests a potential application of product of formula Ia in the treatment of tumors with acquired resistance to camptothecin derivatives.

**Table 0.1**

| Relative resistance of Product of formula Ia and docetaxel against resistant cell lines expressing the P-glycoprotein | | | |
|---|---|---|---|
| **Resistant cell line** | **Resistance factor to** | | ***mdr1*** **mRNA level******* |
| | **Docetaxel** | **Product of formula Ia** | |
| P388/DOX | 50.7 | 10.0 | +++ |
| P388/TXT | 4.8 | 1.8 | ++ |
| P388/VCR | 5.8 | 1.8 | ++ |
| HL60/TAX | 8.1 | 2.5 | ++ |
| Calc18/TXT | 23.5 | 4.3 | ++ |
| KB V1 | 59.0 | 7.6 | ++++ |

| | | | |
|---|---|---|---|
| ** Relative expression obtained from Northern blot experiment using the human mdr1 gene as probe.* | | | |

### CaCo2

The antiproliferative properties of the product of formula Ia and docetaxel were examined on the human colon adenocarcinoma CaCo-2 cell line (ATCC HTB37) that was shown to present a basal level of expression of P-glycoprotein encoded by the *mdr1* gene. The methodology was used, i.e., 96 well plates, time of contact of the drugs (96 hours) and neutral red coloration of viable cell. Results expressed as IC50, were summarized in the next table.

*In vitro* growth inhibitory effect of Product of formula Ia and docetaxel on CaCo-2 cell line

| **Cells** | **Drug** | **IC50 (µg/ml)******* |
|---|---|---|
| CaCo-2 | docetaxel | 108 ± 17 (3) |
| | Product of formula Ia | 22 ± 3 (3) |

| | | |
|---|---|---|
| **Values into brackets: number of experiments.* | | |

Product of formula Ia is cytotoxic on CaCo-2 cells with IC₅₀ included in the range described on the other human cell line tested, *i.e.,* HL60 Calc18 and KB (see previous paragraph). In contrast, docetaxel displays a higher IC₅₀ on CaCo-2 cells, corresponding to 4.9 times the values observed with Product of formula Ia. Therefore, the higher antiproliferative activity of Product of formula Ia, compared to docetaxel, on CaCo-2 cells is in agreement with a lower recognition of Product of formula Ia than docetaxel by the P-glycoprotein intrinsically expressed by this cell line.

### In vivo antitumor activity

*In vivo* schedule of administration and antitumor efficacy studies were carried out using a preclinical standard formulation of Product of formula Ia in ethanol: polysorbate 80 (50:50, v/v). After dilution, the final concentration was 5 % ethanol, 5 % polysorbate, 90 % glucose-5 % in water.

Subsequently, the clinical formulation was developed in which Product of formula Ia was solubilised in polysorbate 80. After dilution, the final polysorbate 80 concentration was 5 %. In the summaries which follow, *in vivo* studies were carried out using the preclinical standard formulation, unless otherwise stated.

Product of formula Ia was administered by the intravenous route (i.v.). The experiments and data analyses were done according to standard protocols.

The activity end points used to assess subcutaneously implanted solid tumors were:
- tumor growth inhibition, T/C, where T and C are the median tumor weights in mg of the treated and the control groups, respectively. According to NCI standards, a T/C > 42 %, -, is inactive, a T/C ≤ 42 %, +, is the minimum level for activity. A T/C < 10 %, ++, is considered as a high antitumor activity level which justifies further development (DN-2 level).

In cases of high antitumor activity, the two following end points were also used:
- tumor growth delay, T-C, where T and C are the median times in days required for the treatment group and the control group tumors to reach a predetermined size (750 to 1500 mg).
- log cell kill, which is the logarithm of the total number of cells killed by treatment. This value can be converted to an arbitrary rating activity according to the Southern Research Institute (SRI) : log cell kill total < 0.7 = - inactive ; 0.7-1.2 = +; 1.3-1.9 = ++ ; 2.0-2.8 = +++ ; >2.8 = ++++ highly active.

For advanced stage tumors, regressions can be either partial (more than 50 % reduction in tumor mass) or complete. Complete regressions are included in the partial regressions.

The end point used for assessing activity in intraperitoneally implanted leukemia was percent increase in host life span (% ILS), calculated in relation to median day of death.

The NCI criteria for activity are as follows:
- P388: highly active ++ ≥ 75 % ILS; active, + 27-74 % ILS; inactive, - < 27 % ILS.

Toxicity was based on drug deaths (≥ 10 %) or a weight loss nadir > 20 %. Docetaxel was used for comparison trials when needed.

### Colon tumors

### Colon 51

Product of formula Ia was administered i.v. on day 4, 6, 8. At the HNTD (9.3 mg/kg/injection), it was found highly active with a 0 % T/C and a 2.6 log cell kill. The dosage below yielded a 2.2 log cell kill. Docetaxel was also found highly active with a 3.1 log cell kill.

Evaluation of i.v. Product of formula Ia against colon adenocarcinoma C51 on BALB/c female mice

| **Agent (batch)** | **Dosage in mg/kg per injection** | **Schedule in days** | **T/C in % day 18** | **T-C in days** | **log cell kill total** | **Comments** |
|---|---|---|---|---|---|---|
| Product of formula Ia | 24.2 | 4,6,8 | - | - | - | Toxic (5/5 DD) |
| | 15.0 | | - | - | - | Toxic (1/5 DD) |
| | 9.3 | | 0 | 25.8 | 2.6 | HNTD active |
| | 5.8 | | 0 | 21.9 | 2.2 | Active |
| Docetaxel | 24.2 | 4,6,8 | - | - | - | Toxic 22.8 % bwl |
| | 15.0 | | 0 | 31.1 | 3.1 | HNTD highly active |
| | 9.3 | | 0 | 22.4 | 2.2 | Active |
| | 5.8 | | 4 | 8.9 | 0.9 | Active |
| Median time for tumor to reach 750 mg in control = 15.1 days. Abbreviations used: HNTD = highest nontoxic dose, bwl = body weight loss, DD = drug death, bwl = body weight loss. | | | | | | |

### Advanced stage colon 38

Product of formula Ia was administered i.v. to mice bearing 200 to 400 mg of tumor. At the HNTD (20 mg/kg/injection), it was found highly active and produced 5/5 complete regressions, all mice being tumor free survivors on day 139. The dosage below (12.4 mg/kg/injection) induced the same number of complete regressions and 80 % tumor free survivors. In comparison, docetaxel at the HNTD produced only 40 % partial regressions in the same trial.

Evaluation of i.v. Product of formula Ia against advanced colon adenocarcinoma C38 on B6D2F₁ female mice

| **Agent (batch)** | **Dosage in mg/kg per injection** | **chedule in days** | **T-C in days** | **log cell kill total** | **Complete regressions** | **Comments** |
|---|---|---|---|---|---|---|
| Product of formula Ia | 32.2 | 14,17,20 | - | - | - | Toxic (1/5 DD) |
| | 20.0 | | - | - | 5/5 | HNTD highly active (5/5 TFS) |
| | 12.4 | | - | - | 5/5 | Highly active (4/5 TFS) |
| | 7.7 | | 25.0 | 2.7 | 2/5 | Active |
| Docetaxel | 32.2 | 14,17,20 | - | - | - | Toxic 20.6% bwl |
| | 20.0 | | 29.1 | 3.1 | 0/5 | HNTD highly active |
| | 12.4 | | 6.1 | 0.7 | 0/5 | Marginal activity |
| | 7.7 | | 2.5 | 0.3 | 0/5 | Inactive |
| Median time for tumor to reach 1000 mg in control = 18.5 days. Abbreviations used: HNTD = highest nontoxic dose, bwl = body weight loss, DD = drug death, TFS = tumor free survivors observed on day 139. | | | | | | |

## Claims

1. Use of a compound of formula (I) for preparing a medecine for treating abnormal cell proliferation of cell lines expressing the multidrug resistance P-glycoprotein, said use comprising administration to a mammal of an effective amount of a compound of formula (I) or a pharmaceutically acceptable salt or solvate. 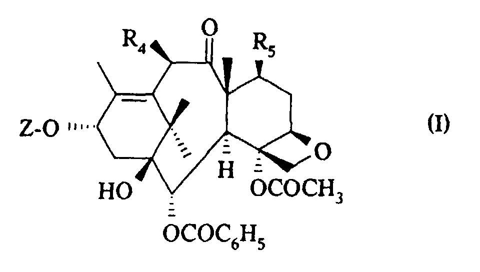 in which:
Z represents a hydrogen atom or a radical of general formula : 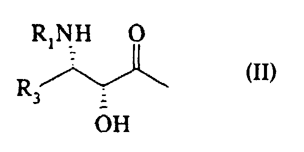 in which :
R₁ represents
a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals,
a thenoyl or furoyl radical or
a radical R₂-O-CO- in which R₂ represents :
an alkyl radical containing 1 to 8 carbon atoms,
an alkenyl radical containing 2 to 8 carbon atoms,
an alkynyl radical containing 3 to 8 carbon atoms,
a cycloalkyl radical containing 3 to 6 carbon atoms,
a cycloalkenyl radical containing 4 to 6 carbon atoms or
a bicycloalkyl radical containing 7 to 10 carbon atoms,
these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or
- a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents
an unbranched or branched alkyl radical containing 1 to 8 carbon atoms,
an unbranched or branched alkenyl radical containing 2 to 8 carbon atoms,
an unbranched or branched alkynyl radical containing 2 to 8 carbon atoms,
a cycloalkyl radical containing 3 to 6 carbon atoms,
a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals,
on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or I- or ϑ-naphthyl radicals,
R₄ represents
an alkoxy radical containing 1 to 6 carbon atoms in an unbranched or branched chain,
an alkenyloxy radical containing 3 to 6 carbon atoms in an unbranched or branched chain,
an alkynyloxy radical containing 3 to 6 carbon atoms in an unbranched or branched chain,
a cycloalkyloxy radical containing 3 to 6 carbon atoms or
a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
R₅ represents
an alkoxy radical containing 1 to 6 carbon atoms in an unbranched or branched chain,
an alkenyloxy radical containing 3 to 6 carbon atoms,
an alkynyloxy radical containing 3 to 6 carbon atoms,
a cycloalkyloxy radical containing 3 to 6 carbon atoms or
a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

2. Use of compound according to claim 1 wherein Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms and alkyl, alkoxy, dialkylamino, acylamino, alkoxycarbonylamino or trifluoromethyl radical, or a 2- or 3-furyl, 2- or 3-thienyl or 2-, 4- or 5-thiazolyl radical, and R₄ and R₅, which may be identical or different, each represent an unbranched or branched alkyloxy radical containing 1 to 6 carbon atoms.

3. Use of a compound according to claim 2 wherein Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R₄ and R₅, which may be identical or different, each represent a methoxy, ethoxy or propoxy radical.

4. Use of 4α-acetoxy-2α-benzoyloxy-5α,20-epoxy-1β-hydroxy-7β,10β-dimethoxy-9-oxo-11-taxen-13α-yl(2R,3S)-3-tert-butoxycarbonylamino-2-hydroxy-3-phenyl-propionate, for preparing a medecine for treating abnormal cell proliferation of cell lines expressing the multidrug resistance P-glycoprotein.

5. Use of compound according to claims 1 to 4 for preparing a medecine usable to treat colon cancer.

6. Use of compound according to claim 5 wherein this use is performed concurrently with at least other therapeutic treatment.

7. Use of compound according to claim 6 wherein the other therapeutic treatment comprises antineoplastic drugs, monoclonal antibodies, immunotherapies, radiotherapies, or biological response modifiers.

8. Use of compound according to claim 1 wherein the medecine is administered by parenteral administration.

9. Use of compound of any of the claims 1 to claim 8 wherein the compound of formula (I) is administered by intravenous, intraperitoneal, intramuscular or subcutaneous administration.
